# EUROPÄISCHE PATENTSCHRIFT

(11) **EP 1 028 129 B1**
(45) Veröffentlichungstag und Bekanntmachung des Hinweises auf die Patenterteilung: **27.04.2005**
(21) Anmeldenummer: 00101919.9
(22) Anmeldetag: 01.02.2000
(51) Int. Cl.: C08F 220/58, C08F 226/02, A61K 47/32, A61K 7/48

(54) **Wasserlösliche oder wasserquellbare Polymerisate**
Water-soluble or water-swellable polymers
Polymères solubles ou expansibles dans l'eau

(30) Priorität: 11.02.1999 DE 19905639
(43) Veröffentlichungstag der Anmeldung: 16.08.2000
(73) Patentinhaber: Clariant GmbH, 65929 Frankfurt am Main (DE)
(72) Erfinder: Brungs, Peter, Dr., 84503 Altötting (DE); Löffler, Matthias, Dr., 65527 Niedernhausen (DE)

(56) Entgegenhaltungen:
- EP-A- 0 510 246
- EP-A- 0 816 403
- EP-A- 0 936 228
- US-A- 4 471 097
- US-A- 4 585 845
- US-A- 4 624 795

## Beschreibung

Die vorliegende Erfindung betrifft die Herstellung wasserlöslicher oder wasserquellbarer Copolymerisate auf Basis von Ammoniumsalzen von Acrylamidoalkylsulfonsäuren und N-Vinylcarbonsäureamiden und deren Verwendung als Verdicker, Stabilisatoren von Emulsionen und Dispersionen und als Gleitmittel in kosmetischen und pharmazeutischen Mitteln zur Einstellung der Viskosität wäßriger Lösungen.

### Stand der Technik

Wasser- oder lösungsmittelhaltige Mehrkomponentensysteme wie Lösungen, Emulsionen oder Suspensionen werden häufig aus ökonomischen oder anwendungstechnischen Gründen oder aus Stabilitätsgründen auf höhere Viskositäten eingestellt bzw. verdickt.
So kann z.B. durch Erhöhung der Viskosität der externen oder internen Phase von Emulsionen oder Suspensionen erreicht werden, daß die Zeit bis zur Entmischung der Komponenten eines solchen Systems deutlich verlängert werden kann, was sich in einer Verlängerung der Lagerzeit bemerkbar macht. Durch Erhöhung der Viskosität wird auch bei vielen Produkten deren gleichmäßige Verteilbarkeit insbesondere auf unebenen Flächen verbessert. Dies gilt insbesondere für Hautpflegemittel und pharmazeutische Salben auf der Haut. Bei vielen technischen Produkten wie Tapetenablösern, Abbeizmitteln oder Flugzeugenteisem verhindert die erhöhte Viskosität ein vorzeitiges Abfließen von der zu behandelnden Fläche. Durch die gleichmäßigere Verteilung und verlängerte Einwirkdauer wird so die Wirksamkeit erhöht. Neben den erwähnten anwendungstechnischen Vorteilen bietet die hohe Viskosität solcher Präparate auch weitere Vorteile bei der Herstellung, Verpackung, Abfüllung und Lagerung sowie beim Transport, insbesondere ist hier aus sicherheitstechnischer Hinsicht die Verdickung saurer Medien von Bedeutung.

Generell sind die rheologischen Eigenschaften bei der Herstellung und/oder Formulierung kosmetischer, pharmazeutischer oder technischer Präparate ein entscheidendes Kriterium für den Einsatz dieser Produkte in der Praxis. Die eingesetzten Verdicker sollen dabei bereits in möglichst geringen Einsatzmengen zu einer ausreichenden Verdickung führen. Jedoch sollen die Farbe und prinzipiellen Eigenschaften des zu verdickenden Mediums nicht verändert werden.

Um die rheologischen Eigenschaften von wäßrigen oder lösungsmittelhaltigen Systemen, Emulsionen, Suspensionen einzustellen, werden in der Fachliteratur eine Vielzahl von unterschiedlichen Systemen angegeben. Bekannt sind beispielsweise Celluloseether und andere Cellulosederivate (z.B. Carboxymethylcellulose, Hydroxyethylcellulose), Gelatine, Stärke und Stärkederivate, Natriumalginat, Fettsäurepolyethylenglykolester, Agar-Agar, Tragant oder Dextrin. Als synthetische Polymere kommen verschiedene Materialien zum Einsatz, wie z.B. Polyvinylalkohole, Polyacrylamide, Polyacrylsäure und verschiedene Salze der Polyacrylsäure, Polyvinylpyrrolidon, Polyvinylmethylether, Polyethylenoxide, Copolymere aus Maleinsäureanhydrid und Vinylmethylether, sowie diverse Mischungen und Copolymerisate aus den o.a. Verbindungen.

Die genannten Verbindungen zeigen jedoch bei der Anwendung vielfältige Nachteile. So sind z.B. die Cellulosederivate bzw. allgemein die auf natürlichen Rohstoffen basierenden Materialien und die daraus resultierenden Formulierungen sehr anfällig gegen Bakterien. Anwendungstechnisch fallen sie zumeist durch die Bildung unangenehmer, fadenziehender" Gele auf. Fettsäurepolyethylenglykolester neigen in Gegenwart von Wasser zur Hydrolyse, die dabei entstehenden unlöslichen Fettsäuren verursachen unerwünschte Trübungen. Verdickungsmittel natürlichen Ursprungs (z.B. Agar-Agar oder Traganth) weisen je nach Herkunft stark schwankende Zusammensetzung auf.

In EP-A-0 816 403 und WO 98/00 094 sind vernetzte Homopolymere aus 2-Acrylamido-2-methyl-propan-sulfonaten und deren Verwendung als Verdicker beschrieben. EP-A-0 510 246 beschreibt unter anderem vernetzte Copolymere aus N-Vinylcarbonsäureamiden und ungesättigten, mit einer Sulfonatgruppe substituierten Alkylamiden, die ebenfalls als Verdicker geeignet sind. Die Sulfonsäuregruppe liegt hierbei ausschließlich in Form der freien Säure vor oder als Alkalimetallsalz.

Überraschenderweise wurde nun gefunden, daß Ammoniumsalze verschiedener Acrylamidoalkylsulfonsäuren, insbesondere das Ammoniumsalz der 2-Acrylamido-2-methyl-propan-sulfonsäure, in für kosmetische Anwendungen unbedenklichen Lösungsmitteln, wie Alkohole oder Alkoholgemische, insbesondere in tert. Butanol, hinreichend löslich sind und somit für eine Copolymerisation mit ebenfalls in diesen Lösungsmitteln oder Lösungsmittelgemischen löslichen N-Vinylcarbonsäureamiden sowie als Vernetzer wirkenden Monomeren hervorragend geeignet sind. Im Gegensatz hierzu muß gemäß dem Stand der Technik in einem aprotischen Lösungsmittel gearbeitet werden. Da das für die Polymerisation eingesetzte Ammoniumsalz der 2-Acrylamido-2-methyl-propan-sulfonsäure in ionischer Form vorliegt, braucht das erhaltene vernetzte Copolymer nicht mehr umständlich neutralisiert zu werden, sondern ist sofort nach Polymerisation und Abtrennung des Lösungsmittels als Verdickungsmittel einsetzbar. Als weiterer Vorteil läßt sich durch das als Comonomer miteingebaute N-Vinylcarbonsäureamid das Verhältnis von ionischen zu neutralen Bausteinen steuern und damit die Verdickungswirkung und Salzstabilität regulieren und speziellen Anforderungen besser anpassen. Durch die Polymerisation in Alkohol bzw. Alkoholgemischen mit einem Wassergehalt von kleiner als 10 Gew.-% und hier insbesondere in tert.-Butanol erhält man außerdem Produkte, die bezüglich ihres Restgehaltes an im Produkt verbleibendem Lösungsmittel toxikologisch unbedenklich sind und so zum Beispiel in kosmetischen Produkten Verwendung finden können.

Die vorliegende Erfindung betrifft wasserlösliche oder wasserquellbare Polymerisate, vorzugsweise mit einer Partikelgröße nicht größer als 10 µm, die neben 0,5 bis 2 Gew.-% vernetzender Strukturen, die aus Monomeren mit mindestens zwei olefinischen Doppelbindungen hervorgegangen sind, in statistischer Verteilung 3 bis 15 Gew.-% der wiederkehrenden Struktureinheit (1) und 84,5 bis 96,5 Gew.-% der wiederkehrenden Struktureinheit (2) enthalten, 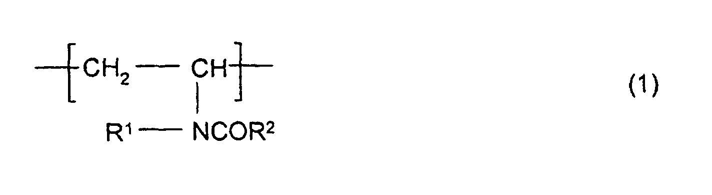 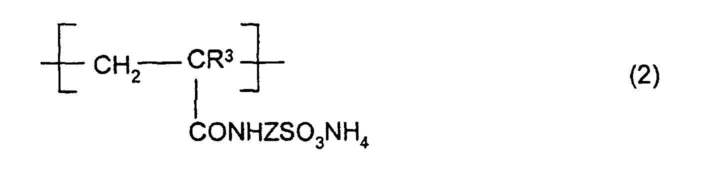 worin R¹, R² und R³ unabhängig voneinander für ein Wasserstoffatom oder eine Methylgruppe stehen und Z für eine C₁-C₄-Alkylengruppe steht.

Vemetzbare Strukturen, die aus Monomeren mit mindestens zwei olefinischen Doppelbindungen hervorgegangen sind, leiten sich vorzugsweise ab von beispielsweise Dipropylenglykol-diallylether, Polyglykoldiallylether, Triethylenglykoldivinylether, Hydrochinon-diallylether, Tetraallyloxyethan oder anderen Allyl- oder Vinylethern, multifunktionellen Alkoholen, Tetraethylenglykoldiacrylat, Triallylamin, Trimethylolpropandiallylether, Methylen-bis-acrylamid oder Divinylbenzol.
Besonders bevorzugt leiten sich die vernetzenden Strukturen ab von Monomeren der allgemeinen Formel (3), 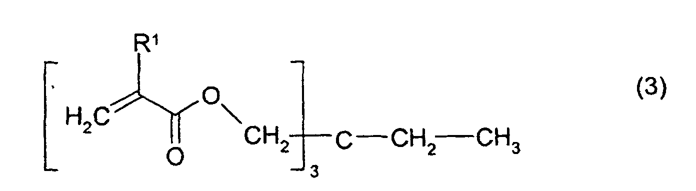 worin R¹ Wasserstoff oder Methyl bedeutet.

Ein bevorzugtes Verfahren zur Herstellung der erfindungsgemäßen Polymerisate besteht darin, daß man zur Herstellung der Polymerisate
a) 84,5 bis 96,5 Gewichtsteile des Ammoniumsalzes einer Acrylamidoalkylsulfonsäure der allgemeinen Formel (4) 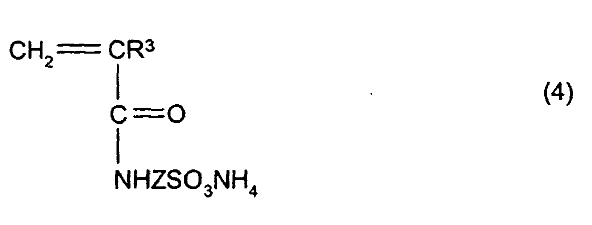 worin R³ und Z die oben angegebenen Bedeutungen haben, in einem alkoholischen Lösungsmittel oder Lösungsmittelgemisch löst oder dispergiert oder 84,5 bis 96,5 Gewichtsteile der aus (4) abgeleiteten freien Acrylamidosulfonsäure in einem alkoholischen Lösungsmittel oder Lösungsmittelgemisch löst oder dispergiert und diese durch Einleiten von Ammoniak bzw. ammoniakalischer Lösung in das Ammoniumsalz überführt,
b) zu der gemäß a) erhaltenen Lösung oder Dispersion 3 bis 15 Gewichtsteile des N-Vinylcarbonsäureamids der allgemeinen Formel (5) zusetzt, 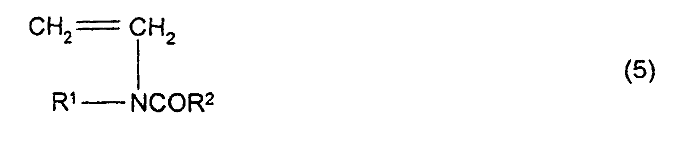 worin R¹ und R² unabhängig die oben angegebenen Bedeutungen haben,
c) zu der gemäß b) erhaltenen Lösung oder Dispersion 0,5 bis 2 Gewichtsteile eines oder mehrerer Vernetzer mit mindestens zwei Doppelbindungen zusetzt, und
d) die Polymerisation in an sich bekannter Weise durch radikalbildende Verbindungen startet und bei einer Temperatur von 10 bis 150 °C durchführt, wobei die Wahl des in a) verwendeten alkoholischen Lösungsmittels oder Lösungsmittelgemisches so erfolgt, daß die erhaltenen Polymerisate weitgehend im Lösungsmittel oder Lösungsmittelgemisch unlöslich sind.

Die Polymerisationsreaktion erfolgt vorzugsweise in einem wasserlöslichen Alkohol oder einem Gemisch mehrerer Alkohole mit 1 bis 4 C-Atomen, vorzugsweise in tert.-Butanol. Der Wassergehalt des Alkohols oder des Gemisches mehrerer Alkohole darf 10 Gew.-% nicht überschreiten, da sonst im Verlauf der Polymerisation Klumpenbildung auftreten kann. Konkret hat die Wahl der Art und der Menge des Lösungsmittels so zu erfolgen, daß die eingesetzte bzw. durch Einleiten von Ammoniak oder ammoniakalischer Lösung generierte Menge an Ammoniumsalz der Acrylamidoalkylsulfonsäure, insbesondere der 2-Acrylamido-2-methyl-propansulfonsäure, weitgehend gelöst oder dispergiert ist. Unter weitgehend gelöst oder dispergiert ist zu verstehen, daß sich auch nach Abstellen des Rührwerks kein festes Material aus der Lösung oder Dispersion absetzt. Das im Verlaufe der Reaktion entstehende Polymerisat soll hingegen in dem gewählten Lösungsmittel (oder - gemisch) weitgehend unlöslich sein. Unter weitgehend unlöslich ist hierbei zu verstehen, daß im Verlauf der Polymerisation eine gut rührbare breiige Polymerpaste entsteht, in der sich keine Klumpen oder Verklebungen bilden dürfen. Das durch Absaugen der Paste erhältliche Filtrat darf einen Feststoffgehalt von maximal 5 Gew.-% aufweisen. Sind die Polymerisate in stärkerem Ausmaß im gewählten Lösungsmittel oder Lösungsmittelgemisch löslich, kann es beim Trocknen der Polymerisatpaste zu Verklumpungen kommen.
Die Polymerisationsreaktion selbst wird in an sich bekannter Weise durch radikalbildende Verbindungen wie Azoinitiatoren (z.B. Azo-bis-isobutyronitril), Peroxiden (z.B. Dilaurylperoxid) oder Persulfaten in einem geeigneten Temperaturintervall von 20 bis 120°C, vorzugsweise zwischen 40 und 80°C, ausgelöst und über einen Zeitram von 30 min. bis mehreren Stunden fortgeführt.

Die Copolymerzusammensetzung läßt sich durch Variation des oben beschriebenen Einsatzverhältnisses der Monomere sowie des Anteils an Vernetzer variieren und so zur Erstellung eines maßgeschneiderten Eigenschaftsprofils verwenden. Durch verstärkten Einbau von Ammoniumsalzen der Acrylamidosulfonsäuren kann beispielsweise die verdickende Wirkung der Polymerisate verbessert werden, während durch Einbau von mehr Gew.-% N-Vinylcarbonsäureamiden die Elektrolytverträglichkeit der Polymerisate und deren Löslichkeit in nicht wäßrigen Systemen verbessert werden kann.
Im Gegensatz zu den Polymerisaten auf Basis Acrylsäure, die im neutralen oder leicht alkalischen Bereich in 1 %iger wäßriger Lösung durchaus Viskositäten von mehr als 30 000 mPa·s zeigen, deren Verdickungsvermögen (bzw. die gemessene Viskosität), jedoch mit sinkendem pH-Wert stark nachläßt, können die erfindungsgemäß beschriebenen Copolymere ihre Viskosität (in 1 %iger wäßriger Lösung) bis zu einem sauren pH-Wert von ca. 3 aufrecht erhalten.

### Beispiel 1:

In einem 1000 ml Kolben mit Ankerrührer, Rückflußkühler, Innenthermometer, Einleitungsmöglichkeit für N₂ und NH₃ wurden 490,5 g tert. Butanol und 11,5 g Wasser vorgelegt. Anschließend wurden 80,75 g 2-Acrylamido-2-methyl-propansulfonsäure eingetragen und unter starkem Rühren dispergiert, wobei eine Trübung des Lösungsmittels erhalten blieb. Über einen Zeitraum von 30 min. leitete man 6,64 g Ammoniak in den überstehenden Gasraum ein und rührte mindestens weitere 30 min. nach bis sich ein pH-Wert von 6-7 eingestellt hatte. Man gab 4,25 g N-Vinylformamid und 1,45 g Trimethylolpropantriacrylat hinzu und spülte die Vorlage jeweils mit tert. Butanol (ca. 6 ml) nach, um Verluste bei der Zugabe zu minimieren. Das Reaktionsgemisch wurde dann auf eine Temperatur von T = 60°C erwärmt, wobei die Reaktionsmischung durch gleichzeitiges Einleiten von N₂ inertisiert wurde. Nach Erreichen der Temperatur von T = 60°C wurden 1,0 g Dilaurylperoxid zugegeben. Die Reaktion sprang unmittelbar nach Zugabe des Initiators an, was an einem Anstieg der Temperatur und am Ausflocken des Polymers zu erkennen war. Etwa 15 min. nach dem Einsetzen der Polymerisationsreaktion wurde die Stickstoffzufuhr abgestellt. Ungefähr 30 min. nach Zugabe des Starters Dilaurylperoxid erreichte die Temperatur ein Maximum (ca. 65 - 70°C). Weitere 30 min. nach Durchlaufen dieses Maximums wurde zum Rückfluß erhitzt und unter diesen Bedingungen zwei Stunden nachgerührt. Der Inhalt des Reaktionsgefäßes nahm im Verlauf der Reaktion eine breiartige Konsistenz an, war aber noch gut rührbar. Anschließend wurde auf Raumtemperatur abgekühlt und der Feststoff abgesaugt. Die Paste wurde bei 60 - 70 °C über 24 h im Vakuumtrockenschrank getrocknet. Man erhielt 92,2 g eines feinen weißen Pulvers.

### Beispiel 2:

Das Beispiel 1 wurde wiederholt mit dem Unterschied, daß anstelle von Trimethylolpropantriacrylat als Vernetzer 1,65 g Trimethylolpropanmethacrylat verwendet wurden.

### Beispiel 3 (Vergleich):

Entsprechend Beispiel 1 wurde das vernetzte Copolymer aus 34 g 2-Acrylamido-2-methyl-propan-sulfonsäure, 51 g N-Vinylformamid und 1,9 g Trimethylolpropantriacrylat hergestellt.

### Beispiel 4 (Vergleich):

Entsprechend Beispiel 1 wurde das vernetzte Copolymer aus 76,5 g 2-Acrylamido-2-methyl-propan-sulfonsäure, 8,5 g N-Vinylformamid und 1,9 g Trimethylolpropantriacrylat hergestellt.

### Testergebnisse:

Die gemäß den Beispielen gewonnenen Pulver wurden jeweils zu 1,0 Gew.-% in destilliertem Wasser aufgelöst und die Viskosität der dabei gebildeten Gele bei 25°C gemessen. Hierzu wurden in einem 600 ml Becherglas jeweils 5 g getrocknetes Polymer-Pulver in 495 g destilliertem Wasser eingerührt und die Viskosität des dabei gebildeten Gels mit einem Brookfield Viskosimeter Typ RVT bei 20 Upm gemessen. Die so hergestellten Gele eignen sich insbesondere für kosmetische Anwendungen, da sie beim Verteilen auf dem Körper ein angenehmes Hautgefühl hervorrufen.

Die Bestimmung der Säurestabilität erfolgte ebenfalls durch Viskositätsmessung mit dem Brookfield-Viskosimeter. Hierzu wurde das nach Herstellungsbeispiel 1 hergestellte Copolymer mit einem handelsüblichen Polymer auf Basis Acrylsäure (Carbopol® 934 der Fa. Goodrich) verglichen. Von beiden Polymeren wurden nach der oben beschriebenen Methode 1,0 %ige Gele hergestellt, deren pH-Wert gegebenenfalls durch Zugabe von NaOH bzw. H₃PO₄ auf einem sauren (pH = ca. 3) und einen neutralen (pH = 6-7) eingestellt wurde.

**Tabelle:**

| gemessene Viskositäten der 1,0 %igen Gele | | |
|---|---|---|
| pH-Wert | Polymer Beispiel 1 | Carbopol 934 (Vergleich) |
| 6 - 7 | 64 200 mPa·s | 76 600 mPa·s |
| ca. 3 | 50 200 mPa·s | 140 mPa·s |

Wie der Tabelle zu entnehmen ist, zeigen im Gegensatz zu den auf Basis von Acrylsäure aufgebauten Polymere die erfindungsgemäß beschriebenen Polymere auch bei sauren pH-Werten sehr gute Verdickungseigenschaften.

Die erfindungsgemäßen Copolymere zeichnen sich aus durch ihre stark verdickende Wirkung, insbesondere in kosmetischen und pharmazeutischen Zubereitungen bei Konzentrationen an festem Copolymer von 0,1 bis 5 Gewichtsprozent, bevorzugt von 0,5 bis 2 Gewichtsprozent, besonders bevorzugt von 0,7 bis 1 Gewichtsprozent bezogen auf das fertige Mittel aus. Bei Raumtemperatur in deionisiertem Wasser werden bei einem pH-Wert von 6 bis 7 Viskositäten von mehr als 60 000 mPa·s erreicht.

Die erfindungsgemäßen Copolymeren zeigen in einem weiten pH-Bereich, insbesondere im Bereich von pH 2,5 bis 7 nur relativ geringe Viskositätsänderungen. Außerdem behalten sie in den Formulierungen ihre gute Wasserlöslichkeit bei und können leicht von der Haut abgewaschen werden. Ihre verdickenden und stabilisierenden Eigenschaften kommen auch in wässrig, alkoholischen und/oder glycolhaltigen Lösungen zur Wirkung. Sie sind UV-stabil und in einem weiten Temperaturbereich von 0°C bis 50°C stabil.

Durch Variation der Monomeren Acrylamidosulfonsäureammoniumsalz und N-Vinylcarbonsäureamid, sowie des Anteils an Vernetzer werden Copolymere erhalten, die sowohl in Öl-in-Wasser Emulsionen, als auch in "Wasser-in-Öl" Emulsionen bei einem pH von 7 bis 2,5 als Verdicker eingesetzt werden können. Abhängig davon, ob Lotionen mit einer vergleichsweise niedrigen oder Cremes und Salben mit hohen Viskositäten hergestellt werden sollen, enthalten Emulsionen einen Ölkörper, bestehend im wesentlichen aus Emulgator/en und einer Ölphase in den Gewichtsmengen von 5 bis 95 %, vorzugsweise 25 bis 85 % und der an 100 Gew.-% fehlenden Menge Wasser. Als Ölkörper kommen pflanzliche, tierische, mineralische und synthetische Öle zum Einsatz, beispielsweise Guerbetalkohole mit 6 bis 18, vorzugsweise 8 bis 10 Kohlenstoffatomen, Ester von linearen C₆-C₁₃-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimerdiol) und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate, Dialkylether und/oder aliphatische bzw. aromatische Kohlenwasserstoffe.

Die Emulsionen können als Hautpflegemittel, wie beispielsweise Tagescremes, Nachtcremes, Pflegecremes, Nährcreme, Bodylotions, Salben und dergleichen vorliegen und als weitere Hilfs- und Zusatzstoffe, Co-Emulgatoren, Überfettungsmittel, Fette, Wachse, Stabilisatoren, biogene Wirkstoffe, Glycerin, Konservierungsmittel, Perlglanzmittel, Farb- und Duftstoffe enthalten.

Als Überfettungsmittel können Substanzen wie beispielsweise polyethoxilierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Typische Beispiele für Fette sind Glyceride, als Wachse kommen u.a. Bienenwachs, Paraffinwachs oder Mikrowachse, gegebenenfalls in Kombination mit hydrophilen Wachsen, z.B. Cetylstearylalkohol in Frage.

Als Stabilisatoren können Metallsalze von Fettsäuren, wie z.B. Magnesium-, Aluminium- und/oder Zinkstearat eingesetzt werden.
Unter biogenen Wirkstoffen sind beispielsweise Pflanzenextrakte und Vitaminkomplexe zu verstehen.
Als Konservierungsmittel eignen sich beispielsweise Phenoxyethanol, Formaldehydlösung, Parabene, Pentandiol oder Sorbinsäure.
Als Perlglanzmittel kommen beispielsweise Glycoldistearinsäureester wie Ethylenglycoldistearat, aber auch Fettsäuremonoglycolester in Betracht. Als Farbstoffe können die für kosmetische Zwecke geeigneten und zugelassenen Substanzen verwendet werden, wie sie beispielsweise in der Publikation Kosmetische Färbemittel" der Farbstoffkommission der Deutschen Forschungsgemeinschaft, Verlag Chemie, Weinheim, 1984, S. 81 - 106, zusammengestellt sind.

Der Gesamtanteil der Hilfs- und Zusatzstoffe kann 1 bis 10, vorzugsweise 2 bis 5 Gew.-%, bezogen auf das Mittel, betragen.
Die Herstellung der Mittel kann in an sich bekannter Weise, d.h. beispielsweise durch Heiß-, Heiß-Heiß/Kalt- bzw. PIT-Emulgierung erfolgen.

Die nachfolgenden Beispiele sollen die Anwendungsmöglichkeiten der erfindungsgemäßen Verdicker näher erläutern, ohne diese darauf einzuschränken. Die Prozentangaben sind in allen Fällen Gewichtsprozente.

### Beispiel 1: O/W-Creme

| | | | |
|---|---|---|---|
| A | Hostacerin DGI | (Clariant GmbH) | 2,00 % |
| | Mineralöl, niedrigviskos | | 8,00 % |
| | Isopropylpalmitat | | 4,00 % |
| | Eutanol G | (Henkel) | 4,00 % |
| | | | |
| B | Copolymer | (Clariant GmbH) | 1,20 % |
| | | | |
| C | Hostapon KCG | (Clariant GmbH) | 0,80 % |
| | Wasser | | ad 100 % |
| | Konservierungsmittel | | q.s. |
| | | | |
| D | Duftstoffe | | 0,40 % |

### Herstellweise

I Einrühren von B in A, dann Hinzufügen von C und gut Verrühren
II Einrühren von D in I
III Homogenisieren

### Beispiel 2: O/W-Hautmilch

| | | | |
|---|---|---|---|
| A | Hostacerin DGMS | (Clariant GmbH) | 2,00 % |
| | Mineralöl, hochviskos | | 8,00 % |
| | Isopropylpalmitat | | 5,00 % |
| | Cetiol 868 | (Henkel) | 4,00 % |
| | | | |
| B | Copolymer | | 0,50 % |
| | | | |
| C | Hostapon KCG | (Clariant GmbH) | 2,00 % |
| | Glycerin | | 4,00 % |
| | Wasser | | ad 100 % |
| | Konservierungsmittel | | q.s. |
| | | | |
| D | Duftstoffe | | 0,30 % |

### Herstellweise:

I Aufschmelzen von A auf ca. 70 °C; Zugabe von B
II Erwärmen von C auf ca. 70 °C
III Einrühren von II in I bis zum Erkalten rühren
IV Zugabe von D bei ca. 35 °C
V Homogenisieren

### Beispiel 3: O/W-Hautmilch

| | | | |
|---|---|---|---|
| A | Hostacerin DGL | (Clariant GmbH) | 2,00 % |
| | Isopropylpalmitat | | 4,00 % |
| | Mandelöl | | 5,00 % |
| | Weizenkeimöl | | 1,00 % |
| | Cetiol SN | (Henkel) | 8,00 % |
| | | | |
| B | Copolymer | | 0,60 % |
| | | | |
| C | Wasser | | ad 100 % |
| | Konservierungsmittel | | q.s. |
| | | | |
| D | Duftstoffe | | 0,30 % |

### Herstellweise:

I A und B mischen und in C einrühren
II D hinzufügen
III Homogenisieren

### Beispiel 4: O/W-Hautmilch

| | | | |
|---|---|---|---|
| A | Hostaphat CG 120 | (Clariant GmbH) | 1,50 % |
| | Mineralöl, niedrigviskos | | 5,00 % |
| | Miglyol 812 | (Dynamit Nobel) | 4,00 % |
| | Isopropylpalmitat | | 6,00 % |
| | Sojaöl | | 3,00 % |
| | Jojobaöl | | 2,00 % |
| | | | |
| B | Copolymer | | 0,80 % |
| | | | |
| C | Hostapon KCG | (Clariant GmbH) | 1,00 % |
| | Wasser | | ad 100 % |
| | Glycerin | | 3,00 % |
| | Soda (10% in Wasser) | | 1,20 % |
| | Konservierungsmittel | | q.s. |
| | | | |
| D | Duftstoffe | | 0,30 % |

### Herstellweise:

I B in A einrühren, C dazugeben und gut vermischen
II D hinzufügen
III Homogenisieren

| Handelsprodukte | | |
|---|---|---|
| ®Hostacerin DGI | (Clariant GmbH) | Polyglyceryl-2-Sesquiisostearat |
| ®Eutanol G | (Henkel) | Octyldodecanol |
| Copolymer | | Copolymer gemäß Beispiel 1 |
| ®Hostapon KCG | (Clariant GmbH) | Natriumcocoylglutamat |
| Hostacerin DGMS | (Clariant GmbH) | Polyglyceryl-2-stearat |
| ®Cetiol 868 | (Henkel) | Octylstearat |
| Hostacerin DGL | (Clariant GmbH) | Polyglyceryl-2 PEG-10 Laurat |
| ®Cetiol SN | (Henkel) | Cetearylisononanoat |
| ®Hostaphat CG 120 | (Clariant GmbH) | Octyldecylphosphat |
| ® Miglyol 812 | (Dynamit Nobel) | Capryltriglycerid |

## Patentansprüche

1. Wasserlösliche oder wasserquellbare Polymerisate, die neben 0,5 bis 2 Gew.-% vernetzenden Strukturen, die aus Monomeren mit mindestens zwei olefinischen Doppelbindungen hervorgegangen sind, in statistischer Verteilung 3 bis 15 Gew.-% der wiederkehrenden Struktureinheit der Formel (1) und 84,5 bis 96,5 Gew.-% der wiederkehrenden Struktureinheit der Formel (2) enthalten, 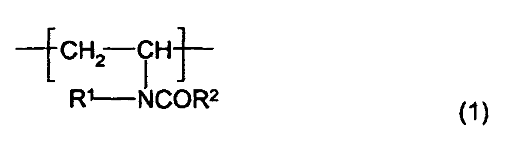 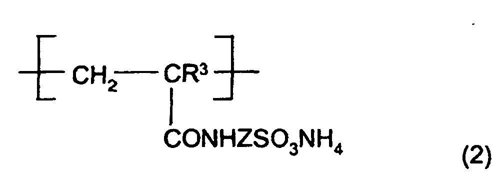 worin R¹, R² und R³ unabhängig voneinander für ein Wasserstoffatom oder eine Methylgruppe stehen und Z für eine C₁-C₄-Alkylengruppe steht.

2. Wasserlösliche oder wasserquellbare Polymerisate gemäß Anspruch 1, **dadurch gekennzeichnet, dass** sich das wiederkehrende Strukturelement der Formel (2) ableitet von dem Ammoniumsalz der 2-Acrylamido-2-methyl-propan-sulfonsäure.

3. Wasserlösliche oder wasserquellbare Polymerisate gemäß Anspruch 1 oder 2, **dadurch gekennzeichnet, dass** sich das wiederkehrende Strukturelement der Formel (1) von N-Vinylformamid ableitet.

4. Wasserlösliche oder wasserquellbare Polymerisate gemäß einem der Ansprüche 1 bis 3, **dadurch gekennzeichnet, dass** das Polymerisat eine durchschnittliche Partikelgröße von 10 µm oder weniger aufweist.

5. Wasserlösliche oder wasserquellbare Polymerisate gemäß einem der Ansprüche 1 bis 4, **dadurch gekennzeichnet, dass** sich die vemetzenden Strukturen ableiten von Dipropylenglykol-diallylether, Polyglykoldiallylether, Triethylenglykoldivinylether, Hydrochinon-diallylether, Tetraallyloxyethan oder anderen Allyl- oder Vinylethern, multifunktionellen Alkoholen, Tetraethylenglykoldiacrylat, Triallylamin, Trimethylolpropan-diallylether, Methylen-bis-acrylamid oder Divinylbenzol.

6. Wasserlösliche oder wasserquellbare Polymerisate gemäß einem der Ansprüche 1 bis 5, **dadurch gekennzeichnet, dass** sich die vernetzenden Strukturen ableiten von Monomeren der allgemeinen Formel (3), 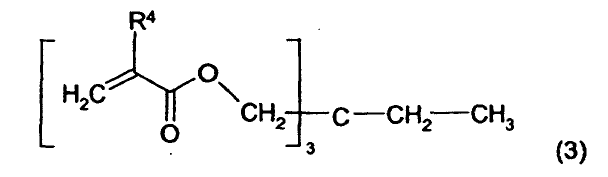 worin R⁴ Wasserstoff oder Methyl bedeutet.

7. Verfahren zur Herstellung der wasserlöslichen oder wasserquellbaren Polymerisate gemäß einem oder mehrerer der Ansprüche 1 bis 6, **dadurch gekennzeichnet, dass** man
a) 84,5 bis 96,5 Gewichtsteile des Ammoniumsalzes der Acrylamidoalkylsulfonsäure der allgemeinen Formel (4), 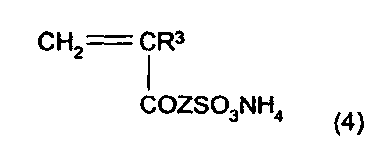 worin R³ und Z die oben angegebenen Bedeutungen haben in einem alkoholischen Lösungsmittel oder Lösungsmittelgemisch löst oder dispergiert, oder 84,5 bis 96,5 Gewichtsteile der aus (4) abgeleiteten freien Acrylamidosulfon-säure in einem alkoholischen Lösungsmittel löst oder dispergiert und diese durch Einleiten von Ammoniak bzw. ammoniakalischer Lösung in das Ammoniumsalz überführt,
b) zu der gemäß a) erhaltenen Lösung oder Dispersion 3 bis 15 Gewichtsteile des N-Vinylcarbonsäureamids der allgemeinen Formel (5) 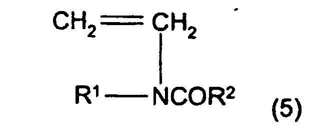 worin R¹ und R² die in Anspruch 1 angegebenen Bedeutungen haben, zusetzt,
c) zu der gemäß b) erhaltenen Lösung oder Dispersion 0,5 bis 2 Gewichtsteile eines oder mehrerer Vernetzer mit mindestens zwei Doppelbindungen zusetzt, und
d) die Polymerisation in an sich bekannter Weise durch radikalbildende Verbindungen startet und bei einer Temperatur von 10 bis 150 °C durchführt, wobei die Wahl des in a) verwendeten alkoholischen Lösungsmittels oder Lösungsmittelgemischs so erfolgt, dass die erhaltenen Polymerisate weitgehend im Lösungsmittel oder Lösungsmittelgemisch unlöslich sind.

8. Verwendung der Polymerisate gemäß einem oder mehrerer der Ansprüche 1 bis 6 zum Verdicken von Flüssigkeiten.

9. Verwendung der Polymerisate gemäß einem oder mehrerer der Ansprüche 1 bis 6 zum Verdicken von kosmetischen und pharmazeutischen Zubereitungen.

10. Verwendung der Polymerisate gemäß der Ansprüche 8 oder 9, **dadurch gekennzeichnet, dass** diese in den Gewichtsmengen von 0,1 bis 5 Gew.-%, bevorzugt von 2 bis 0,5 Gew.-%, besonders bevorzugt von 0,7 bis 1 Gew.-% eingesetzt werden.

11. Verwendung der Polymerisate gemäß der Ansprüche 8 oder 9 in einem pH-Bereich von pH 2,5 bis 7, bevorzugt im pH-Bereich 3,0 bis 5,0.

## Claims

1. A water-soluble or water-swellable polymer which, besides from 0.5 to 2% by weight of crosslinking structures originating from monomers having at least two olefinic double bonds, comprises, in random distribution, from 3 to 15% by weight of the repeat structural unit of the formula (1) and from 84.5 to 96.5% by weight of the repeat structural unit of the formula (2), 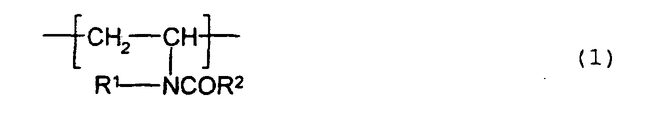 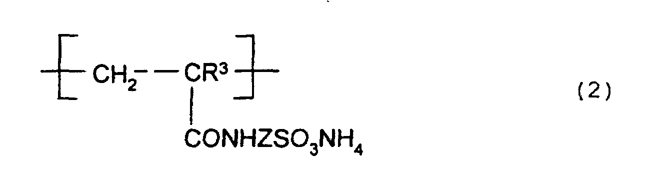 in which R¹, R² and R³ independently of one another are a hydrogen atom or a methyl group, and Z is a C₁-C₄-alkylene group.

2. The water-soluble or water-swellable polymer as claimed in claim 1, wherein the repeat structural element of the formula (2) is derived from the ammonium salt of 2-acrylamido-2-methylpropanesulfonic acid.

3. The water-soluble or water-swellable polymer as claimed in claim 1 or 2, wherein the repeat structural element of the formula (1) is derived from N-vinylformamide.

4. The water-soluble or water-swellable polymer as claimed in any of claims 1 to 3, wherein the polymer has an average particle size of 10 µm or less.

5. The water-soluble or water-swellable polymer as claimed in any of claims 1 to 4, wherein the crosslinking structures are derived from dipropylene glycol diallyl ether, polyglycol diallyl ether, triethylene glycol divinyl ether, hydroquinone diallyl ether, tetraallyloxyethane or other allyl or vinyl ethers, polyfunctional alcohols, tetraethylene glycol diacrylate, triallylamine, trimethylolpropane diallyl ether, methylenebisacrylamide or divinylbenzene.

6. The water-soluble or water-swellable polymer as claimed in any of claims 1 to 5, wherein the crosslinking structures are derived from monomers of the formula (3), 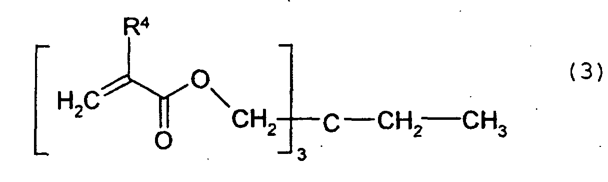 in which R⁴ is hydrogen or methyl.

7. A process for the preparation of the water-soluble or water-swellable polymers as claimed in one or more of claims 1 to 6, which comprises
a) dissolving or dispersing from 84.5 to 96.5 parts by weight of the ammonium salt of acrylamidoalkylsulfonic acid of the formula (4), 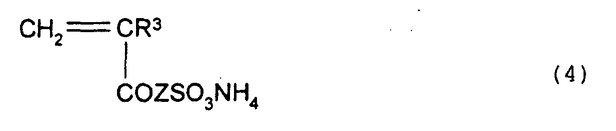 in which R³ and Z are as defined above, in an alcoholic solvent or solvent mixture, or dissolving or dispersing from 84.5 to 96.5 parts by weight of the free acrylamidosulfonic acid derived from (4) in an alcoholic solvent and converting the acid into the ammonium salt by introducing ammonia or ammoniacal solution,
b) adding to the solution or dispersion obtained as in a) from 3 to 15 parts by weight of the N-vinylcarboxamide of the formula (5) 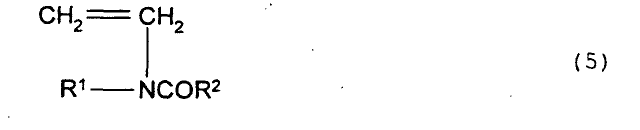 in which R¹ and R² are as defined in claim 1,
c) adding to the solution or dispersion obtained as in b) from 0.5 to 2 parts by weight of one or more crosslinkers having at least two double bonds, and
d) starting the polymerization in a manner known per se using free-radical-forming compounds, and carrying out the polymerization at a temperature of from 10 to 150°C, the alcoholic solvent or solvent mixture used in a) being chosen such that the resulting polymers are largely insoluble in the solvent or solvent mixture.

8. The use of the polymers as claimed in one or more of claims 1 to 6 for thickening liquids.

9. The use of the polymers as claimed in one or more of claims 1 to 6 for thickening cosmetic and pharmaceutical preparations.

10. The use of the polymers as claimed in claims 8 or 9, wherein the polymers are used in amounts by weight of from 0.1 to 5% by weight, preferably from 2 to 0.5% by weight, particularly preferably from 0.7 to 1% by weight.

11. The use of the polymers as claimed in claims 8 or 9 in a pH range from pH 2.5 to 7, preferably in a pH range from 3.0 to 5.0.

## Revendications

1. Polymères solubles dans l'eau ou pouvant gonfler dans l'eau, qui outre 0,5 à 2 % en poids de structures réticulables qui dérivent de monomères ayant au moins deux doubles liaisons oléfiniques, contiennent selon une distribution statistique 3 à 15 % en poids du motif structural répétitif de formule (1) et 84,5 à 96,5 % en poids du motif structural répétitif de formule (2) 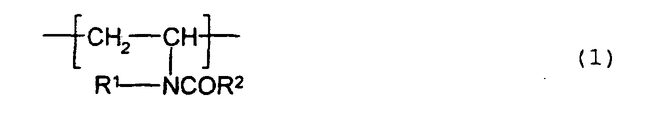 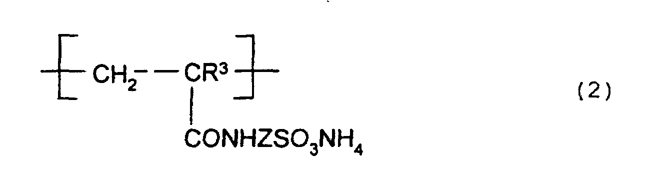 dans lesquels R¹, R² et R³ représentent chacun indépendamment des autres un atome d'hydrogène ou un groupe méthyle, et Z est un groupe alkylène en C₁-C₄.

2. Polymères solubles dans l'eau ou pouvant gonfler dans l'eau selon la revendication 1, **caractérisés en ce que** l'élément structural répétitif de formule (2) dérive du sel d'ammonium de l'acide 2-acrylamido-2-méthylpropanesulfonique.

3. Polymères solubles dans l'eau ou pouvant gonfler dans l'eau selon la revendication 1 ou 2, **caractérisés en ce que** l'élément structural répétitif de formule (1) dérive du N-vinylformamide.

4. Polymères solubles dans l'eau ou pouvant gonfler dans l'eau selon l'une des revendications 1 à 3, **caractérisés en ce que** le polymère présente une granulométrie moyenne de 10 µm ou moins.

5. Polymères solubles dans l'eau ou pouvant gonfler dans l'eau selon l'une des revendications 1 à 4, **caractérisés en ce que** les structures réticulables dérivent de l'éther diallylique du dipropylèneglycol de l'éther diallylique du polyglycol, de l'éther divinylique du triéthylèneglycol, de l'éther diallylique de l'hydroquinone, du tétraallyloxyéthane ou d'autres éthers allyliques ou vinyliques, d'alcools multifonctionnels, du diacrylate de tétraéthylèneglycol, de la triallylamine, de l'éther diallylique du triméthylolpropane, du méthylène-bisacrylamide ou du divinylbenzène.

6. Polymères solubles dans l'eau ou pouvant gonfler dans l'eau selon l'une des revendications 1 à 5, **caractérisés en ce que** les structures réticulables dérivent de monomères ayant la formule générale (3) 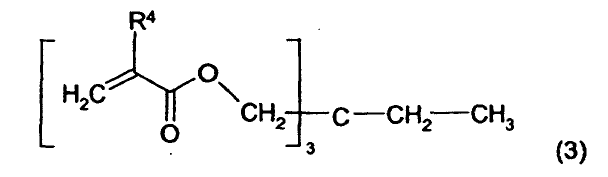 dans laquelle R⁴ est un atome d'hydrogène ou le groupe méthyle.

7. Procédé de préparation des polymères solubles dans l'eau ou pouvant gonfler dans l'eau selon l'une ou plusieurs des revendications 1 à 6, **caractérisé en ce que**
a) on dissout ou disperse 84,5 à 96, 5 parties en poids du sel d'ammonium de l'acide acrylamidoalkylsulfonique de formule générale (4) 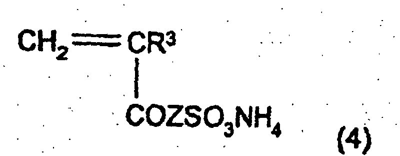 dans laquelle R³ et Z ont les significations données ci-dessus, dans un solvant alcoolique ou un mélange de solvants alcooliques, ou encore on dissout ou on disperse 84,5 à 96,5 parties en poids de l'acide acrylamidosulfonique libre dérivé de (4) dans un solvant alcoolique, et on le convertit, par introduction d'ammoniac ou d'une solution ammoniacale, en le sel d'ammonium,
b) à la solution ou dispersion obtenue en a), on ajoute 3 à 15 parties en poids du N-vinylcarboxamide de formule générale (5) 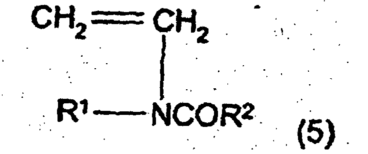 dans laquelle R¹ et R² ont les significations données dans la revendication 1 ;
c) à la solution ou dispersion obtenue en b), on ajoute 0,5 à 2 parties en poids d'un ou plusieurs agents de réticulation ayant au moins deux doubles liaisons, et
d) on lance la polymérisation d'une manière connue en soi à l'aide d'amorceurs radicalaires, et on la met en oeuvre à une température de 10 à 150°C, le choix du solvant ou du mélange de solvants alcooliques utilisés en a) étant réalisé de telle sorte que les polymères obtenus soient pour ainsi dire insolubles dans le solvant ou dans le mélange de solvants.

8. Utilisation des polymères selon l'une ou plusieurs des revendications 1 à 6 pour épaissir des liquides.

9. Utilisation des polymères selon l'une ou plusieurs des revendications 1 à 6 pour épaissir des préparations cosmétiques et pharmaceutiques.

10. Utilisation des polymères selon la revendication 8 ou 9, **caractérisée en ce que** ces derniers sont utilisés en des quantités pondérales de 0,1 à 5 % en poids, de préférence de 2 à 0,5 % en poids et plus particulièrement de 0,7 à 1 % en poids.

11. Utilisation des polymères selon les revendications 8 ou 9 dans une plage de pH de 2,5 à 7 et de préférence de 3,0 à 5,0.
